Europäisches Patentamt

European Patent Office

Office européen des brevets

(11) Publication number: **0 420 170 A2**

# EUROPEAN PATENT APPLICATION

(21) Application number: **90118443.2**

(22) Date of filing: **26.09.90**

(51) Int. Cl.5: **G01N 33/543, G01N 33/569**

(30) Priority: **28.09.89 US 414161**

(43) Date of publication of application:
**03.04.91 Bulletin 91/14**

(84) Designated Contracting States:
**AT BE CH DE DK ES FR GB GR IT LI LU NL SE**

(71) Applicant: **Becton Dickinson and Company**
**One Becton Drive**
**Franklin Lakes New Jersey 07417-1880(US)**

(72) Inventor: **Hoke, Randal A.**
**107 Steel trap Cort**
**Cary, North Carolina(US)**
Inventor: **Nycz, Colleen M.**
**6412 Pernod way**
**Raleigh, North Carolina(US)**
Inventor: **Robson, Jillian A.**
**46 Fearrington Poast**
**Pittsboro, North Carolina(US)**

(74) Representative: **von Kreisler, Alek,**
**Dipl.-Chem. et al**
**Patentanwälte Von Kreisler-Selting-Werner,**
**Deichmannhaus am Hauptbahnhof**
**W-5000 Köln 1(DE)**

(54) **Stabilizing reagent for membrane immunoassay.**

(57) A method for immunoassay for a ligand is performed on a porous membrane wherein the signal detected is development of a color on the membrane. The color of the signal and background is substantially stopped and stabilized for reading of the assay at a later time by passing a color stabilizing reagent which includes an acid through the membrane.

EP 0 420 170 A2

## STABILIZING REAGENT FOR MEMBRANE IMMUNOASSAY

## BACKGROUND OF THE INVENTION

### 1. Field of the Invention.

This invention relates to immunoassay for an analyte, and more particularly relates to membrane immunoassay and particular reagents useful therein.

### 2. Background of the Invention.

Assay systems which are both rapid and sensitive have been developed to determine the concentration of a substance, generally referred to as the analyte, present in low concentration in a fluid sample. Immunoassays depend on the binding of an antigen or hapten to a specific antibody and have been particularly useful because they give high levels of specificity and sensitivity. These assays employ one of the above reagents in labeled form, the labeled reagent being referred to as the tracer.

Enzymes have often been used as labels in immunoassay. In conventional enzyme immunoassay (EIA), an enzyme is covalently conjugated with one component of a specifically binding antigen-antibody pair, and the resulting enzyme conjugate is reacted with a substrate to produce a signal which is detected and measured. The signal may be a color change, detected with the naked eye or by a spectrophotometric technique, or may be conversion of the substrate to a product detected by fluorescence.

A convenient format for EIA is solid phase immunoassay in which one of the assay reagents is immobilized on a solid support. The solid support may be in the form of a dipstick, the inside wall of a test tube or cuvette or the well of a microtiter plate. A particularly useful solid support is a microporous membrane.

Membrane immunoassay is often referred to as flow-through assay. Examples of flow-through EIA wherein flow is generated by capillary action are the assays described in U.S. Patent No. 3,888,629 to Bagshaw, U.S. Patent No. 4,246,339 to Cole et al. and U.S. Patent No. 4,632,901 to Valkirs et al. U.S. Patent No. 4,277,560 to Gray and U.S. Patent No. 4,812,293 to McLaurin et al. are examples of flow-through assays using pressure and vacuum respectively.

In membrane EIA, any number of liquids may be caused to flow-through the membrane to effect binding, separation and washing of assay components. The final step in most membrane EIA procedures is passage of a color developing reagent, such as a chromogen, through the membrane. The chromogen reacts with enzyme captured on the membrane to produce a color change which may be detected as evidence of the presence of analyte or measured as evidence of the concentration of analyte.

A problem encountered in most colorimetric membrane EIA procedures heretofore disclosed is impermanence of the contrast between the color developed due to analyte and the background color. Immediate reading of the assay before color fades or overdevelops, or background color approaches that due to analyte is often required. A method to stabilize the contrast would be a valuable advance in the membrane EIA art, particularly where multiple assays are being performed or when it is desired to read the assay at a later time. The present invention provides a reagent and method to overcome this problem.

## SUMMARY OF THE INVENTION

A flow-through assay method for determining a ligand suspected to be present in a liquid includes passing the liquid and a tracer comprising an enzyme through a membrane coated with an inert protein whereby the ligand attaches to the membrane and binds to the tracer. In another embodiment of the method, the membrane additionally has coated thereon an antiligand which binds specifically to the ligand. In the present disclosure, the term inert protein means a protein which is immunologically unreactive toward any other component of the assay and which does not substantially bind nonspecifically to other proteins in the assay medium, with the understanding that the inert protein may well be immunologically reactive toward other materials which are not part of the assay of the invention. After binding, the membrane is separated from the liquid and the enzyme on the membrane is contacted with a substrate comprising an indoxyl derivative which is converted to a colored insoluble product which precipitates as a visible spot on the membrane. The contrast between the color of the spot and the background color is stabilized for later viewing by passing a color stopping and stabilizing solution, hereinafter referred to as the stabilizer, which includes an acid through the membrane.

Preferred ligands are viral antigens detected by an assay format in which the enzyme component of the tracer is conjugated to a specific antibody. Preferred enzymes are hydrolases such as β-galactosidase and alkaline phosphatase and preferred substrates additionally include a tetrazolium salt.

The stabilizer may be any of a variety of mineral or preferably organic acids, the most preferred of which is citric acid, and may include an organic solvent such as an alcohol.

The invention includes a kit of materials useful in performing the assay of the invention.

Thus, the invention provides a flow-through EIA in which the color of the spot generated on the membrane due to the ligand and the background color are stabilized for later interpretation. Multiple assays may be performed without interruption for reading, and a series of assays performed at different times on samples from a given patient may be simultaneously compared to follow the progress of an administered medication.


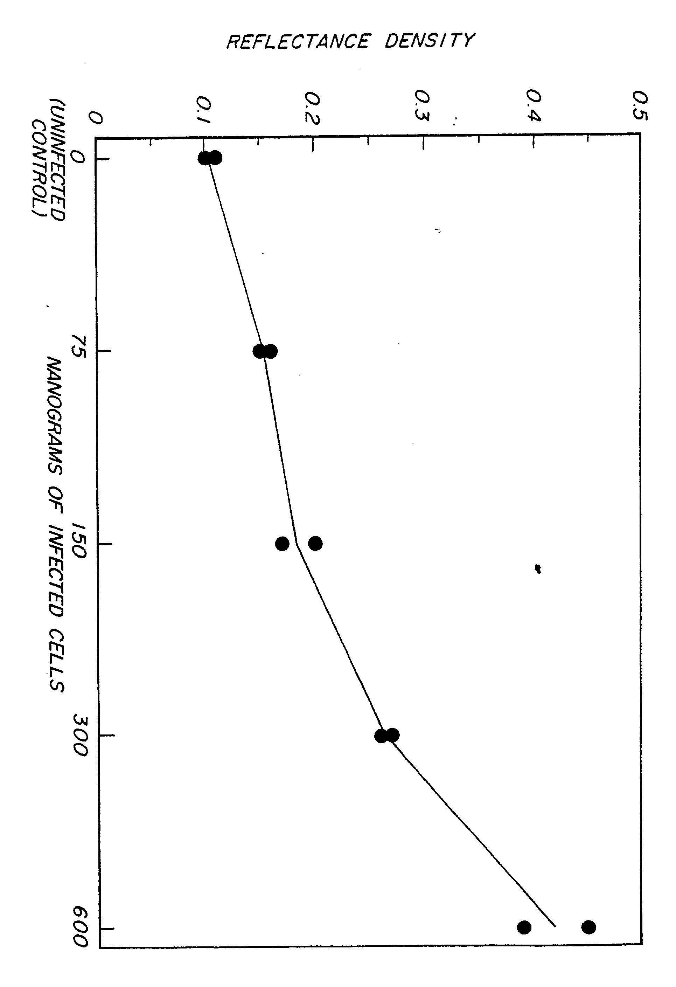

BRIEF DESCRIPTION OF THE DRAWING


The Figure shows the results of an assay for Influenza virus in accordance with the assay of the invention.

DETAILED DESCRIPTION


While this invention is satisfied by embodiments in many different forms, there will herein be described in detail preferred embodiments of the invention, with the understanding that the present disclosure is to be considered as exemplary of the principles of the invention and is not intended to limit the invention to the embodiments illustrated and described. The scope of the invention will be measured by the appended claims and their equivalents.

One aspect of the present invention is a method for colorimetric flow-through immunoassay of a ligand in a liquid in which the assay signal is a colored precipitate deposited on a membrane. Applicants have discovered that the contrast between the color and the background color may be stabilized by treatment with a particular stabilizer so as to remain substantially unchanged for up to 24 hours or longer thereby making it possible to read the assay result at a later time. In contrast, prior art flow-through assays require immediate reading because the contrast is not stable.

The assay of the invention may be performed in any suitable assay device adapted for flow-through assay as known in the art. In preferred devices, flow of assay liquids is promoted by capillary action induced by a pad of absorbent material positioned below the membrane. Representative of preferred devices is the ICON™ device described in U.S. Patent No. 4,632,901, supra.

The ligand may be from any source, and may be an antigen, an antibody or a hapten. For example, the ligand may be an antigen present in a body fluid, or it may be isolated from a body fluid and subsequently introduced into a different liquid, such as buffer. In other cases, the ligand may be from a source other than a body fluid, as, for example, a culture of microorganisms such as Chlamydia or a cellular extract thereof. Preferred ligands are antigens, most preferably viral antigens present in a body fluid, such as Adenovirus, Parainfluenza 3 virus and, most preferably, Herpes simplex virus (HSV), Respiratory syncytial virus (RSV), and Influenza A (Flu A). The invention will hereinafter be described generically in terms of a viral antigen.

Turning now to a detailed description of the assay components, the porous membrane may be of any material which does not interfere in any way with any other component or step of the assay. Suitable membranes are, for example, of glass fiber, polyvinylidene difluoride, polycarbonate, nitrocellulose and nylon. Such membranes are well-known in the art and many are commercially available from suppliers such as Pall, Glen Cove, New York, Millipore, Bedford, Massachusetts, and Schleicher and Schuell, Keene, New Hampshire.

The membrane may be coated with an antiligand specific for the ligand. Thus, in the case where the ligand is the preferred viral antigen, the antiligand may be an antibody which binds specifically to the antigen and thereby captures the antigen on the membrane. The membrane may be further coated with an

3

inert protein to fill any binding sites on the membrane not occupied by the capture antibody. Representative nonlimiting examples of suitable inert proteins are casein and albumin, although others will be evident to those skilled in the art. Coating of both the inert protein and the antibody to the membrane may be carried out by any suitable method, preferably by incubating the membrane with a solution of the protein whereby the protein is physically absorbed into the polymeric matrix of the surface of the membrane.

In a preferred embodiment of the invention, the membrane is coated with the inert protein, the antigen absorbed directly onto this surface and the assay performed without a capture antibody. Flow-through immunoassay absent a capture antibody is disclosed in copending application serial number 272,380, filed November 17, 1988, of common assignee herewith.

The membrane having a coating of capture antibody and/or inert protein is exposed to the sample suspected of containing the viral antigen. Preferably, the coated membrane is incubated with the sample in a transient, flow-through format for about 1 to 15, preferably about 5 minutes at a temperature of about 0 to 50°C, preferably about ambient temperature. By this procedure, antigen in the sample is captured on the coated membrane in proportion to its concentration in the sample. In addition, it has been found that viral antigen is absorbed preferentially even when the sample contains a large excess of extraneous protein, such as is the case when the sample is a body fluid.

The tracer comprises an enzyme conjugated to either the antigen or a detection antibody. Any enzyme may be used which may be conjugated to the antigen or antibody and for which a substrate convertible to a colored product exists. Suitable enzymes are, for example, hydrolases such as peptidases, esterases, phosphatases and glycosidases. The most preferred tracers include alkaline phosphatase or carboxyesterase. Conjugation of enzymes to antigens or antibodies is well-known and fully understood by those skilled in the art.

The choice of substrate of course depends on the enzyme component of the tracer and a wide variety of substrates are well-known for each class of enzymes. Preferred substrates are those which form insoluble precipitates on membranes. Suitable substrates for hydrolases are indoxyl derivatives. For example, when the enzyme is $\beta$-galactosidase, a preferred substrate is indoxyl-$\beta$-D-galactopyranoside. When the enzyme is alkaline phosphatase, a "preferred substrate is indoxyl phosphate. When the enzyme is an esterase, preferred substrates are indoxyl acetate and butyrate. Other indoxyl substrates as known in the art may be used, as for example, 5-bromo-4-chloro-indoxyl derivatives. An extensive list of indoxyls useful for preparation of suitable indoxyl derivatives by standard methods is given by Holt et al., Proceedings of the Royal Society of London, B , 1958, 148 , 481-494. The substrate may preferably be dissolved in water, saline or a suitable buffer.

It is preferred that the color resulting from the cleavage of the indoxyl substrate be augmented by inclusion of a tetrazolium salt in a substrate composition. Suitable tetrazolium salts are, for example, p-iodonitrotetrazolium violet (INT) and nitroblue tetrazolium (NBT). Although a colored spot of sufficient intensity for visualization develops in the absence of the tetrazolium salt, inclusion of this reagent gives a spot of deeper color which may be more easily visualized and may accordingly increase the sensitivity of the assay.

The assay of the invention may be performed by either the competitive or sandwich technique. In the competitive assay of the invention, the tracer is the antigen having the enzyme conjugated thereto wherein the antigen and the tracer compete for available binding sites on the coated membrane. In the preferred assay method of the invention, the coated membrane having viral antigen captured thereon is incubated with a solution of the tracer in a sandwich format to induce immunological binding of the antigen and the detection antibody. Alternatively, the coated membrane may be incubated with antigen and tracer simultaneously. The membrane may then be separated from the liquid phase of the assay medium by causing the liquid to pass through the membrane. Liquid flow through the membrane may be by gravity or preferably may be enhanced by capillary action induced by absorbent material positioned under the membrane. The substrate composition may then be passed through the membrane. Enzyme on the membrane converts the substrate to a product detectable by color. The extent of color formation is proportional to antigen concentration, which may be determined by assaying liquid samples having predetermined quantities of antigen therein and comparing color intensities.

In colorimetric flow-through assays of the prior art, color continues to develop on the membrane as long as enzyme and unreacted substrate are present. In accordance with the invention, the color forming reaction may be stopped and the color on the membrane substantially stabilized by passing a solution of the stabilizer through the membrane. Further, the gradual increase in background color over time, which is often seen in colormetric membrane assays and which may lead to difficulty in distinguishing a positive signal from background unless the signal is read immediately, is substantially stabilized.

Suitable stabilizers in accordance with the invention are mineral acids such as hydrochloric, sulfuric,

4

phosphoric and pyrophosphoric acids. A preferred stabilizer is a solution comprising an aqueous solution of an organic acid, optionally containing an organic solvent. Suitable organic acids are, for example, acetic, tartaric, oxalic succinic, benzoic and preferably citric acids. Suitable organic solvents are methanol, ethanol, isopropanol, acetone and tetrahydrofuran. The concentration of the organic acid may be about 0.1 to 0.5, preferably about 0.1 to 0.2 molar in water or buffer which may optionally contain about 30 to 70, preferably about 45 to 55% by weight of the organic solvent. The solution preferably has a pH of about 0 to 4, most preferably about 2 to 3.5 and accordingly may contain sufficient base, such as sodium hydroxide, to achieve the desired pH.

Another aspect of the invention is a reagent kit or package of materials for performing an assay for a ligand in accordance with the method of the invention. The kit may include a membrane coated with an inert protein and optionally with a capture antiligand, a tracer comprising an enzyme conjugated to one of the ligand or a detection antiligand, a substrate for the enzyme and a stabilizer comprising a solution of an acid, preferably an aqueous solution. The acid solution may be combined with a solvent. The kit may also include standards for the ligand, as, for example, one or more ligand samples of known concentration, or it may include other reagents, enzyme substrates, or solutions, such as saline or buffers useful in carrying out the assay. The membrane may be provided in a housing, preferably plastic, containing a material positioned under the membrane, such as absorbent paper, to facilitate flow of assay liquids through the membranes by capillary action.

The following examples are provided to further describe the invention but are not to be considered in any way as limitative of the invention.

## EXAMPLE 1

### Assay for Respiratory Syncyctial Virus (RSV)

A membrane filter stack was assembled with the following configuration:
Top layer - Three micron Immunodyne Immunoaffinity Membrane, (Pall, Glen Cove, New York, #BIA0030HC5). Precoated by immersion in phosphate buffered saline containing 0.3% casein for 30 minutes at ambient temperature.
Next layer - Non-woven rayon sheet (Schleicher and Schuell, Keene, New Hampshire; #5-5).
Bottom layer - Cellulose absorbent pads (2) (Filtration Sciences, Mount Holly Springs, Pennsylvania; #ED 320-200)

The membrane layers were encased in a plastic holder which includes a receiving well formed above the top layer. Within this well was fitted a flow restriction insert which has an aperture more narrow than the receiving well and sits flush against the top membrane.

An antigen stock was prepared with respiratory syncytial virus (RSV) (Long strain) infected HEp-2 cells diluted in a buffer containing: 250 mM tris(hydroxymethyl)aminomethane hydrochloride (Tris HCl), 150 mM sodium chloride (NaCl), 10 mM ethylene-diaminetetraacetate (EDTA), 4% (v/v) polyoxyethylene sorbitan monolaurate (Tween 20), 1% N-acetyl cysteine, 0.2% sodium azide (NaN₃), pH 8.5. Control antigen was prepared in a similar manner from uninfected HEp-2 cells.

A 150 µL aliquot of this antigen (or control) was applied to the device and allowed to drain through the flow restriction insert and onto the top membrane layer. (Liquid is drawn through the top membrane by the capillary action of the supporting absorbent layers.) The flow restriction insert was then removed, and to the device was added 150 µL of a wash solution consisting of 50 mM Tris HCl, 150 mM NaCl, 0.2% NaN₃, pH 7.2 (Tris buffered saline (TBS)), additionally containing 1 mg/mL of rabbit IgG.

A solution containing 27 µg/mL of anti-RSV antibody conjugated to alkaline phosphatase was prepared in a buffer containing 50 mM Tris HCl, 100 mM NaCl, 200 mM sodium phosphate, 1% casein, 1 mM magnesium chloride, 0.1 mM zinc chloride, and 1 mM 2-mercaptoethanol, pH 7.5. A 150µL aliquot of this mixture was added to the device and allowed to absorb into the membrane stack. Following a brief (two minute) incubation, the device was washed with 300 µL of TBS (without IgG).

A 150 µL solution containing 0.33 mg/mL nitroblue tetrazolium, 1% methanol, and 0.2% NaN₃ was added to the device. This was followed by the addition of 150 µL of a solution containing 2 mg/mL of indoxyl phosphate, 12 mM levamisole in 50 mM 2-amino-2-methyl-1-propanol (AMP) acetate, 0.2% NaN₃, and 19 mM magnesium chloride, at pH 9.8. Following a five minute incubation at ambient temperature, the color forming reaction was stopped by the addition of 150 $\mu$L of a solution prepared from 100 mM sodium citrate, 0.4% benzoic acid, pH 3.3, mixed with various volumes of ethanol (0, 20, 40, 60 80% final containing 5% 2-propanol and 5% methanol as denaturants.) The devices were illuminated with common fluorescent lights and read after 72 hours. The following observations were noted:

The devices treated with 0, 20 and 40% ethanolic stop solutions showed a darkening of the background (the area surrounding the interior signal area).

The device treated with 60% ethanolic stop solution had a light background, but showed some blue/green color just at the edge of the membrane (at the contact point between membrane and plastic top).

The device treated with 80% ethanolic solution had a light background, but the blue/green edge color was more pronounced than in the "60%" device.

A second experiment was performed in which citrate concentration was varied (100, 200, 300 mM) as well as ethanol concentration (40, 45, 50, 55, 60%) in the following grid:

| | | Final ethanol concentration (%) | | | | |
|---|---|---|---|---|---|---|
| | | 40 | 45 | 50 | 55 | 60 |
| Citrate concentration (mM) prior to ethanol addition | 100 | A | B | C | D | E |
| | 200 | F | G | H | I | J |
| | 300 | K | L | M | N | O |

Again, the devices were illuminated by fluorescent lights for approximately 72 hours. The following observations were noted:

The devices treated with 100 mM citrate/ethanol (A through E) had noticeably greenish backgrounds compared with 200, 300 mM citrate (F through O). This was especially true for devices treated with the 55, 60% ethanol (D through E).

The 100 mM citrate/40% ethanol sample (A) had a much darker background than the other devices.

The best result was obtained with a solution prepared by mixing 300 mM sodium citrate, pH 3.1, containing 0.4% by weight of benzoic acid with an equal volume of ethanol wherein a purple-gray spot representing the assay signal was surrounded by a light yellow background.

EXAMPLE 2

Assay for Influenza Virus

This example used the filter stack and plastic holder of Example 1 except the membrane was a 3 micron Biodyne C membrane (Pall, catalog number BNPCH5).

An antigen stock was prepared with type A influenza virus (Flu-A) (WSN strain) infected Madin-Darby canine kidney (MDCK) cells diluted in a buffer containing: 250 mM Tris HCl, 10mM EDTA, 1 mM ethylenebis(oxyethylenenitrilo)tetraacetate (EGTA), 4% (v/v) Tween 20, 1% N -acetyl-L -cysteine, 0.2% sodium azide (NaN$_3$), pH 8.5. Control antigen was prepared in a similar manner from uninfected MDCK cells.

A 150 $\mu$L aliquot of this antigen (or control) was applied to the device and allowed to drain through the flow restriction insert and onto the top membrane layer. The flow restriciton insert was then removed, and to the device was added 150 $\mu$L of TBS, additionally containing 1 mg/mL of rabbit IgG.

A solution containing 27 $\mu$g/mL of anti-Flu-A antibody conjugated to alkaline phosphatase was prepared in a buffer containing 100 mM Tris HCl, 150 mM NaCl, 200 mM sodium phosphate, 1% casein, 1 mM magnesium chloride, 0.1 mM zinc chloride, 1 mM 2-mercaptoethanol, and 0.2% NaN$_3$, pH 7.2. A 150 $\mu$L aliquot of this mixture was added to the device and allowed to absorb into the membrane stack. Following a brief (two minute) incubation, the device was washed with 300 $\mu$L of TBS (without IgG).

A 150$\mu$L solution containing 0.33 mg/mL nitroblue tetrazolium, 1% methanol, and 0.2% NaN$_3$ was

6

added to the device. This was followed by the addition of 150 μL of a solution containing 2 mg/mL indoxyl phosphate, 16 mM levamisole in 50 mM AMP acetate, 0.2% NaN₃, 1 mM magnesium chloride, at pH 9.8. Following a five minute incubation at ambient temperature, the color forming reaction was stopped by the addition of 150 μL of a stabilizing solution containing 150 mM sodium citrate at pH 3.0. The relative color density of the resulting signal spots was measured with a reflectance densitometer (Gretag, Seattle, Washington, model 183). The results of an experiment performed with a series of antigen dilutions are presented in the Figure.

EXAMPLE 3

Comparison of Acidic Stabilizing Reagents

In this Example, the membrane filter stack and plastic holder were as described in Example 2.

An antigen stock was prepared with Herpes - Simplex virus (type II) infected Vero cells diluted in a buffer containing: 250 mM Tris HCl, 150 mM sodium chloride (NaCl), 10 mM EDTA 4% (v/v) Tween 20, 1% N -acetyl-L -cysteine, 0.2% sodium azide (NaN₃), pH 8.5. Control antigen was prepared in a similar manner from uninfected Vero cells.

A 150 μL aliquot of this antigen (or control) containing 120 ng of material was applied to the device and allowed to drain through the flow restriction insert and onto the top membrane layer. This was followed by the addition of 300 μL of 15% hydrogen peroxide in 50 mM TBS. The flow restriction insert was then removed, and to the device was added 150 μL of a TBS wash solution additionally containing 1 mg/mL of rabbit IgG.

A solution containing 40 μg/mL of an anti-HSV antibody conjugated to alkaline phosphatase was prepared in a buffer containing 50 mM Tris HCl, 100 mM NaCl, 200 mM sodium phosphate, 1% casein, 1 mM magnesium chloride, 0.1 mM zinc chloride, and 1 mM 2-mercaptoethanol, pH 7.5. A 150 μL aliquot of this mixture was added to the device and allowed to absorb into the membrane stack. Following a brief (two minute) incubation, the device was washed with 300 μL of TBS (without IgG).

A 150 μL solution containing 0.33 mg/mL nitroblue tetrazolium, 1% methanol, and 0.2% NaN₃ was added to the device. This was followed by the addition of 150 μL of a solution containing 2 mg/mL indoxyl phosphate, 20 mM levamisole in 50 mM AMP acetate, 0.2% NaN₃, 1 mM magnesium chloride, at pH 9.8. Following a five minute incubation at ambient temperature; the color forming reaction was stopped by the addition of 150 μL of the following stabilizing solutions.

1. No stabilizing reagent
2. Water
3. 4.5 N Sulfuric Acid
4. 1 N Hydrochloric Acid
5. 150 mM Hydrochloric Acid
6. 150 mM Citric Acid, pH 3.0
7. 150 mM Citric Acid, pH 1.9
8. 150 mM Citric Acid, pH 3.5
9. 150 mM Citric Acid, pH 5.0
10. 150 mM Citric Acid, pH 6.5
11. 150 mM Phosphoric Acid, pH 2.5
12. 150 mM Pyrophosphoric Acid, pH 2.5
13. 150 mM Acetic Acid, pH 2.5
14. 150 mM Oxalic Acid, pH 2.5
15. 150 mM Glycine, pH 2.5

The relative color density of the resulting signal spots and background areas was measured with the densitometer immediately after addition of the stabilizing solution (0 hours) and again at times of 1/2, 1 1/2, 4 and 24 hours. The results of this experiment are presented in the Table and are in arbitrary units of reflectance.

7

TABLE

| TIME hr. STABILIZER | 0 | | 0.5 | | 1.5 | | 4 | | 24 | |
|---|---|---|---|---|---|---|---|---|---|---|
| | B* | S** | B | S | B | S | B | S | B | S |
| 1 | 0.153 | 0.773 | 0.450 | 1.537 | 0.893 | 1.513 | 1.313 | 1.503 | 1.123 | 1.200 |
| 2 | 0.077 | 0.577 | 0.147 | 0.780 | 0.260 | 0.893 | 0.637 | 1.030 | 0.953 | 1.190 |
| 3 | 0.133 | 0.547 | 0.103 | 0.550 | 0.093 | 0.523 | 0.083 | 0.510 | 0.070 | 0.373 |
| 4 | 0.067 | 0.540 | 0.103 | 0.593 | 0.087 | 0.533 | 0.110 | 0.543 | 0.153 | 0.487 |
| 5 | 0.085 | 0.615 | 0.155 | 0.605 | 0.135 | 0.600 | 0.155 | 0.595 | 0.635 | 0.835 |
| 6 | 0.113 | 0.650 | 0.120 | 0.583 | 0.120 | 0.573 | 0.190 | 0.577 | 0.377 | 0.653 |
| 7 | 0.130 | 0.627 | 0.110 | 0.597 | 0.100 | 0.593 | 0.143 | 0.597 | 0.313 | 0.643 |
| 8 | 0.083 | 0.643 | 0.107 | 0.640 | 0.123 | 0.643 | 0.197 | 0.657 | 0.390 | 0.703 |
| 9 | 0.061 | 0.540 | 0.147 | 0.680 | 0.113 | 0.643 | 0.217 | 0.667 | 0.740 | 0.883 |
| 10 | 0.190 | 0.757 | 0.143 | 0.727 | 0.233 | 0.753 | 0.600 | 0.887 | 0.920 | 1.103 |
| 11 | 0.210 | 0.690 | 0.117 | 0.630 | 0.097 | 0.613 | 0.123 | 0.607 | 0.703 | 0.880 |
| 12 | 0.163 | 0.690 | 0.130 | 0.657 | 0.130 | 0.633 | 0.147 | 0.623 | 0.713 | 0.840 |
| 13 | 0.167 | 0.703 | 0.133 | 0.657 | 0.117 | 0.637 | 0.140 | 0.637 | 0.803 | 1.030 |
| 14 | 0.153 | 0.707 | 0.183 | 0.700 | 0.197 | 0.680 | 0.320 | 0.697 | 0.553 | 0.743 |
| 15 | 0.130 | 0.727 | 0.153 | 0.720 | 0.140 | 0.717 | 0.160 | 0.707 | 0.733 | 0.870 |

\*    Background

\*\*   Signal

It is readily seen that when no stabilizer (entry 1) or water alone (entry 2) are added to the membrane, both signal color and background color continue to develop, and after 4 hours are substantially indistinguishable. In contrast, the acid stabilizers of the invention, when used at pH of 4 or less, stabilize both signal and background. When the preferred citric acid stabilizer is used at weakly acidic pH (entries 9 and 10), poor stabilization results compared to the same stabilizer at pH below 4, (entries 6,7,8).

**Claims**

1. A method for determining a ligand in a liquid comprising:

a) combining a first liquid suspected of containing a ligand with a porous membrane coated with an inert protein and with a tracer including a hydrolase whereby said ligand attaches to the coated membrane and to said tracer to give a bound fraction including said enzyme on said membrane;

b) separating said membrane from said first liquid by causing said first liquid to pass through said membrane;

c) passing a second liquid containing a substrate for said hydrolase comprising a tetrazolium salt and an indoxyl derivative through said membrane, said substrate being converted by said hydrolase on said membrane to a colored product;

d) directing a color stabilizing reagent comprising an acid through said membrane; and

e) determining the presence of said ligand in said first liquid by detecting said colored product on said membrane.

2. The method of Claim 2 wherein said ligand is a viral antigen selected from the group consisting of Herpes simplex virus, Respiratory syncytial virus and Influenza A virus.

3. The method of Claim 1 wherein said tracer further comprises an antiligand specific for said ligand having said hydrolase conjugated thereto, said hydrolase being selected from the group consisting of a peptidase, esterase, phosphatase and glycosidase.

4. The method of Claim 1 wherein said acid is selected from the group consisting of a mineral acid and an organic acid.

5. The method of Claim 1 wherein said reagent further comprises an organic solvent.

6. A method for determining a viral antigen in a liquid comprising:

a) combining a first liquid suspected of containing a viral antigen with a membrane coated with an inert protein and with an antibody specific for said antigen having alkaline phosphatase conjugated thereto whereby said viral antigen attaches to said coated membrane and to said antibody to give a bound fraction including said alkaline phosphatase on said membrane;

b) separating said membrane from said first liquid by causing said first liquid to pass through said membrane;

c) passing a second liquid containing indoxyl phosphate and nitroblue tetrazolium through said membrane, said alkaline phosphatase converting said indoxyl phosphate and nitroblue tetrazolium to a colored product on said membrane;

d) directing a solution comprising citric acid through said membrane; and

e) determining the presence of said antigen in said first liquid by detecting said colored product on said membrane.

7. A kit of materials for performing an assay for a ligand in a liquid comprising a membrane coated with at least one of an inert protein and a first antiligand, a tracer comprising a hydrolase conjugated to one of said ligand and a second antiligand, a substrate for said enzyme and a color stabilizing reagent comprising an acid.

8. The kit of Claim 16 wherein said substrate comprises a derivative of indoxyl.

9. The kit of Claim 19 wherein said substrate further comprises a tetrazolium salt.

10. The kit of Claim 16 further comprising a housing containing an absorbent material positioned adjacent said membrane.

REFLECTANCE DENSITY

0.1  0.2  0.3  0.4  0.5

0

0

75

150

300

600

NANOGRAMS OF INFECTED CELLS

(UNINFECTED CONTROL)